Europäisches Patentamt

European Patent Office

Office européen des brevets

(11) Veröffentlichungsnummer: **0 029 479**

**B1**

(19)

(12) **EUROPÄISCHE PATENTSCHRIFT**

(45) Veröffentlichungstag der Patentschrift: **28.12.83**

(51) Int. Cl.³: **A 61 N 1/36**

(21) Anmeldenummer: **80103513.0**

(22) Anmeldetag: **23.06.80**

(54) Einrichtung zur elektrischen Stimulation des Herzens.

(30) Priorität: **23.11.79 DD 217097**

(43) Veröffentlichungstag der Anmeldung:
**03.06.81 Patentblatt 81/22**

(45) Bekanntmachung des Hinweises auf die Patenterteilung:
**28.12.83 Patentblatt 83/52**

(84) Benannte Vertragsstaaten:
**AT CH DE FR LI NL SE**

(56) Entgegenhaltungen:
**DD - A - 113 439**
**DD - A - 138 034**
**DE - A - 2 747 730**
**DE - A - 2 748 973**

(73) Patentinhaber: **VEB Transformatoren- und
Röntgenwerk "Hermann Matern"
Overbeckstrasse 48
DDR-8030 Dresden (DD)**

(72) Erfinder: **Igel, Gerald, Dipl.-Ing.
Elisabethstrasse 52
DDR-1140 Berlin (DD)**
Erfinder: **Tostmann, Reiner, Dipl.-Phys.
Eichenallee 13
DDR-1424 Leegebruch (DD)**
Erfinder: **Kraft, Diethart, Dr.sc.-techn.
Davidstrasse 1
DDR-7010 Leipzig (DD)**
Erfinder: **Günther, Kurt, Dr.sc.-techn.
Marchwitzastrasse 6
DDR-7030 Leipzig (DD)**

(74) Vertreter: **Beetz, sen., Richard, Dipl.-Ing.
Patentanwälte Dipl.-Ing. R. Beetz sen. Dipl.-Ing.
K. Lamprecht, Dr.Ing. R. Beetz jr. et al,
Rechtsanwalt Dipl.-Phys. Dr. jur. U. Heidrich, Dr.-
Ing. W. Timpe Dipl.-Ing. J. Siegfried Priv.-Doz.
Dipl.-Chem. Dr.rer.nat. W. Schmitt-Fumian
Steinsdorfstrasse 10 D-8000 München 22 (DE)**

Courier Press, Leamington Spa, England.

## Einrichtung zur elektrischen Stimulation des Herzens

Die Erfindung bezieht sich auf eine Einrichtung zur elektrischen Stimulation des Herzens mit einem elektronischen Impulserzeuger und einer Reizelektrode, insbesondere einer Ösophaguselektrode.

Kommt es zu lebensbedrohlichen Störungen des Herzrhythmus oder gar zum Herstillstand, so ist es notwendig, daß möglichst unverzüglich eine Stimulation des Herzens erfolgt, um mit Erfolg den Herzrhythmus zu regulieren bzw. natürliche Herzaktionen hervorzurufen.

Es sind eine Reihe von Herzdiagnose- und Therapiegeräten bekannt, z.B. stationäre EKG-Überwachungs- und Stimulationsgeräte, die bei bedrohlichen Störungen der Herztätigkeit von Patienten in klinischen Einrichtungen zur externen Stimulation angewendet werden. Diese komplexen Geräte sind entweder mit Elektroden zur Impulsabgabe ausgerüstet, die dem Patienten auf die Brust aufgelegt werden oder mit einer transvenös einschwemmbaren endocardialen Elektrode bzw. einer Ösophaguselektrode und einer auf der Brust angeordneten zweiten Elektrode. Nachteilig sind bei derartigen universell einsetzbaren Geräten ihr komplizierter Aufbau und die hohen Anschaffungskosten. Außerdem erfordert die Bedienung und Überwachung eines solchen Gerätes sowohl eine technisch als auch eine medizinisch geschulte Person. Aufgrund des großen apparatemäßigen und personellen Aufwandes sind diese Geräte meistens nur in speziellen klinischen Einrichtungen vorhanden, so daß sie bei auftretenden Notfallsituationen außerhalb dieser Einrichtungen nicht zur Verfügung stehen.

Zur elektrischen Stimulation des Herzens in Notfallsituationen ist eine mit einer Ösophaguselektrode und ihrer indifferenten Elektrode ausgestattete batteriebetriebene Einrichtung bekannt (DD—PS 138 034). Bei dieser, insbesondere für die Behandlung lebensbedrohlicher bradykarder Rhythmusstörungen und bei Herstillstand anwendbaren Einrichtung arbeitet ein als astabiler Multivibrator ausgebildeter Taktgeber mit zwei umschaltbaren Starrfrequenzen über einen Leistungsverstärker auf einen Transformator, über den die Stimulationsimpulse an die Ösophaguselektrode geführt werden. Diese auf ein Minimum an Bauelementen reduzierte Stimulationseinrichtung kann allerdings erhöhten Anforderungen an eine Spannungs- und Formkonstanz der Ausgangsimpulse, vor allem bei Änderung der Patientenimpedanz und bei Absinken der Versorgungsspannung nicht voll genügen.

Der Erfindung liegt die Aufgabe zugrunde, eine Einrichtung zur Stimulation des Herzens in Notfallsituationen zu schaffen, die auch bei Änderung der Patientenimpedanz und Absinken der Versorgungsspannung elektrische Impulse hinreichend großer Ausgangsspannung und Stromstärke sowie genügender Formgenauigkeit und zeitlicher Konstanz über eine Reizelektrode, insbesondere eine Ösophaguselektrode zur Verfügung Stellt und darüber hinaus eine hohe Patientensicherheit bei Ausfällen von Bauelementen bietet sowie einen geringen Energieverbrauch hat.

Diese Aufgabe wird erfindungsgemäß durch die im Anspruch 1 angegebenen Merkmale gelöst. Als Gleichspannungswandler eignet sich insbesondere ein Sperrwandler, da die am Ausgang der Stimulationseinrichtung benötigte Energie nicht auf einmal übertragen werden muß, sondern sie wird portionsweise während der Stimulationsimpulspausen übertragen, gespeichert, und während der Stimulatinsimpulsdauer an den Ausgang abgegeben. Es ist möglich, den Impulserzeuger so auszubilden, daß dieser R-Wellen synchronisiert arbeitet. Eine weitere mögliche Ausführungsform sieht vor, daß der Impulserzeuger bei einer natürlichen Herzaktion blockierbar ist. Um den Bedienenden darüber zu informieren, wann Stimulationsimpulse an die Reizelektrode abgegeben werden, ist vorzugsweise eine Signalisierungsschaltung vorgesehen. Aufgrund dessen, daß sich die Stimulationseinrichtung nicht ständig im Einsatz befindet, ist es vorteilhaft, wenn zur Kontrolle eine weitere Signalisierungsschaltung, z.B. eine Blinkschaltung, vorgesehen ist, die den Ladezustand der Spannungsquelle anzeigt. Der Sperrwandler besteht vorzugsweise aus einem astabilen Multivibrator, einer Leistungsstufe, einem Transformator, einem Gleichrichter und einem Kondensator. Zur Regelung der Ladespannung am Kondensator ist es möglich, ein Dämpfungsglied, welches mit dem astabilen Multivibrator verknüpft ist, vorzusehen, d.h. die Regelung der Ladespannung erfolgt durch Variation des Tastverhältnisses der Sperrwandlerfrequenz. Eine sichere Regelung der Ladespannung am Kondensator wird dadurch erreicht, daß ein Schwellwertschalter und ein bistabiler Multivibrator vorgesehen sowie zwischen astabilen Multivibrator und Leistungsstufe ein UND-Glied eingeschaltet sind, wobei die Ladespannung dem Schwellwertschalter zugeführt ist, der set-Eingang des bistabilen Multivibrators mit dem Ausgang des Schwellwertschalters, der reset-Eingang mit einem Ausgang des Impulserzeugers und der Ausgang des bistabilen Multivibrators mit einem Eingang des UND-Gliedes verknüpft sind. Dies bedeutet, daß das Tastverhältnis der Sperrwandlerfrequenz unverändert bleibt, jedoch mit Erreichen des Sollwertes der Ladespannung durch Sperren des UND-Gliedes keine Energie mehr übertragen wird und zwar bis zum Ende des nächsten Stimulationsimpulses oder bei Verwendung des negierten Stimulationsimpulses bis zum Beginn desselben. Eine weitere vorteilhafte Ausbildungsform der Erfindung sieht vor, daß zwischen Reizelektrode und Kondensator eine Diode geschaltet ist und daß der gleiche Belag des Kondensators mit einer zweiten, entgegengesetzt gepolten Diode verbunden ist, wobei die Entladung des Kondensators über die erstgenannte Diode und die Reizelektrode, die Aufladung des Kondensators hingegen über die zweite Diode erfolgt. Hierdurch und dadurch, daß zur Ausgangsspannungs-begrenzung eine Überlastungsschutzschaltung, z.B. eine Reihenschaltung von Zenerdioden, vorgesehen ist, welcher eine der Ladespannung des Kondensators proportionale Größe zugeführt ist, wird eine höhere Sicherheit für den Patienten bei Ausfällen von Bauelementen gewährleistet. Schließlich soll ein zwischen die Reizelektrode und die Diode geschalteter Widerstand verhindern, daß bei einem Kurzschluß zwischen den Elektrodenanschlüssen der elektronische Schalter zerstört wird.

Die durch die Erfindung erreichten Vorteile sind im wesentlichen darin zu sehen, daß es gelungen ist, eine handliche, an beliebigen Orten anwendbare und von einem Arzt ohne kardiologische Spezialausbildung bedienbare Einrichtung anzugeben, die bei einem verhältnismäßig geringen Aufwand an Bauelementen gegenüber der vergleichbaren, beschriebenen bekannten Einrichtung erhöhten Anforderungen dahin gehend genügt, daß auch bei Änderung der Patientenimpedanz und Absinken der Versorgungsspannung elektrische Impulse hinreichend großer Ausgangsspannung und Stromstärke sowie genügender Formgenauigkeit und zeitlicher Konstanz zur Verfügung gestellt werden. Darüber hinaus wird eine hohe Patientensicherheit bei Ausfällen von Bauelementen gewährleistet. Der Energieverbrauch ist gering.

Im folgenden wird die Erfindung anhand von lediglich einen Ausführungsweg darstellenden Zeichnungen näher erläutert.

Es zeigen

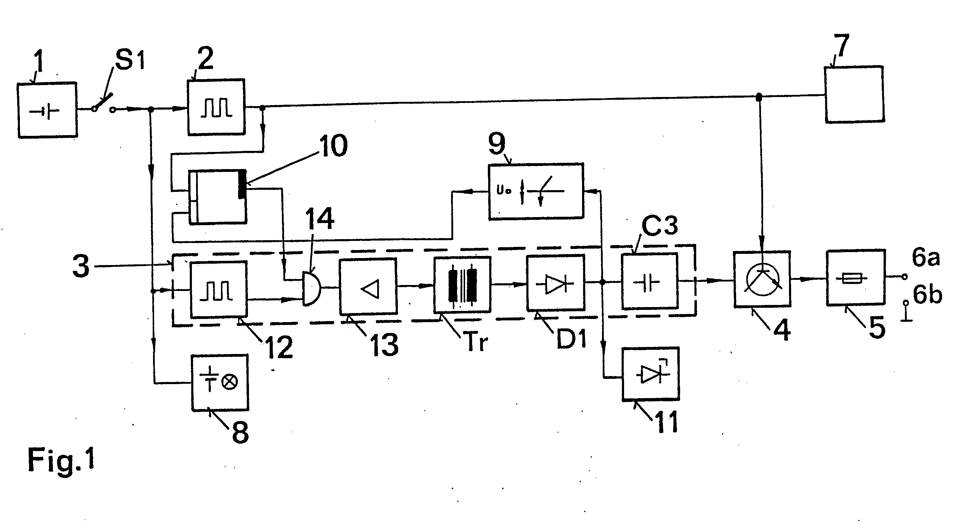

Fig. 1 das Blockschaltbild einer erfindungsgemäßen Einrichtung zur elektrischen Stimulation des Herzens in Notfallsituationen mit Hilfe einer Ösophaguselektrode und

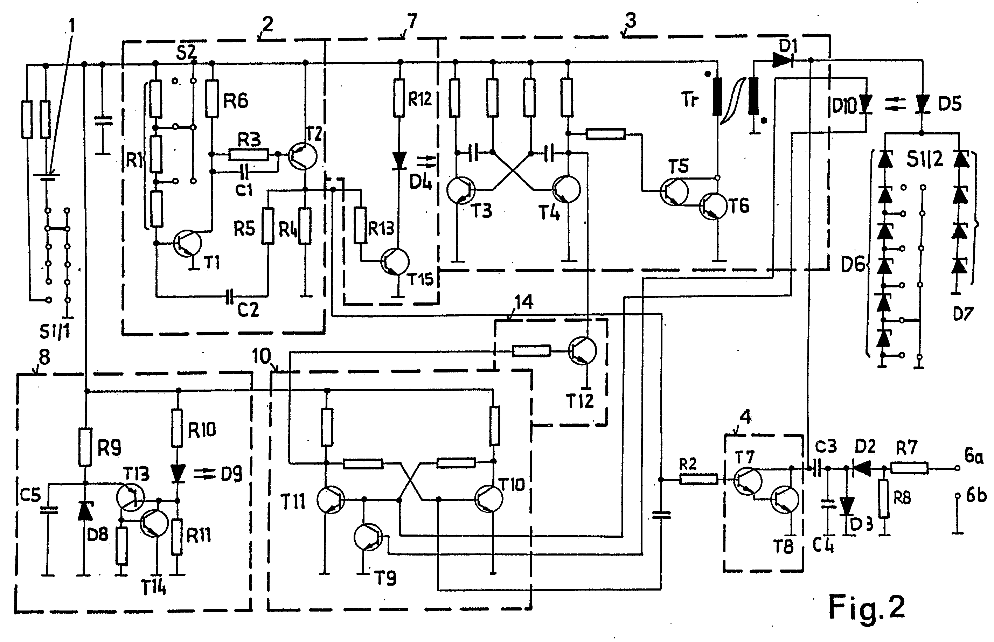

Fig. 2 die komplette Schaltungsanordnung dieser Einrichtung.

Die Einrichtung zur Stimulation des Herzens in Notfällen besitzt als Spannungsquelle 1 eine Batterie, die eine Gleichspannung von 9 V zur Verfügung stellt. Diese Gleichspannung wird über einen Stufenschalter S1 sowohl einem Impulserzeuger 2 als auch einem Gleichspannungswandler 3, in diesem Fall einem Sperrwandler zugeführt, der seinerseits die erforderliche hohe Ausgangsspannung von maximal 90 V bereitstellt. Mit Hilfe eines elektronischen Schalters 4 wird für die vom Impulserzeuger 2 bestimmte Zeitdauer des Stimulationsimpulses die Ausgangsspannung vom Gleichspannungswandler 3 über eine Sicherheitsschaltung 5 an die Ösophaguselektrode geschaltet. Die Sicherheitsschaltung 5 verhindert, daß bei Ausfall von Bauelementen im Patienten gefährliche Impulse appliziert werden bzw. über den Patienten ein Gleichstrom fließt. Parallel zu den Ausgangsbuchsen 6a; 6b für den Anschluß der Ösophaguselektrode liegt ein Widerstand R 8, der eine stetige Belastung des elektronischen Schalters gewährleistet. Eine Signalisierungsschaltung 7 zeigt an, wann Stimulationsimpulse an die Ösophaguselektrode abgegeben werden. Eine weitere Signalisierungsschaltung 8 informiert den Bedienenden über den Ladezustand der Spannungsquelle 1. Bei Blinken einer Kontrollanzeige hat die Batteriespannung einen Wert erreicht, bei dem sich die Kenngrößen der Stimulationsimpulse nicht mehr in dem zu garantierenden Bereich befinden. Zur Stabilisierung der Ausgangsspannung des Gleichspannungswandlers 3 dient eine Regelkreis, dem ein Schwellwertschalter 9, ein bistabiler Multivibrator 10 sowie ein UND-Glied 14 angehören. Dem Ausgangskreis des Gleichspannungswandlers 3 ist schließlich eine Überlastungsschutzschaltung 11 zugeordnet, die bei einem Defekt bestimmter Bauelemente wirksam wird.

Nachfolgend werden die einzelnen Baugruppen und deren Zusammenschaltung näher beschrieben.

Der von der Spannungsquelle 1 gespeiste Impulserzeuger 2 ist ein astabiler Multivibrator bestehend aus zwei Transistoren T1 und T2, zwei Kondensatoren C1; C2, einem frequenzbestimmenden Widerstand R1 und weiteren Widerständen R2 bis R6. Der frequenzbestimmende Widerstand R1 ist als Reihenschaltung aus drei Teilwiderständen ausgebildet, die mit Hilfe eines Stufenschalters S2 kurzgeschlossen werden können. Der astabile Multivibrator erzeugt Impulse von ca. 1,5 ms Dauer mit einer Folgefrequenz von 70, 90 bzw. 110 Impulse pro Minute, wobei die Impulsfrequenz am Stufenschalter S2 eingestellt wird und die Impulse über den Widerstand R2 dem elektronischen Schalter 4 zugeführt werden. Es ist auch denkbar, anstatt eines starrfrequent einstellbaren Impulserzeigers 2 einen solchen zu verwenden, der mit der Herzfrequenz synchronisierbar oder der bei einer natürlichen Herzaktion blockbierbar ist.

Der Gleichspannungswandler 3 besteht aus einem astabilen Multivibrator 12, einer Leistungsstufe 13, einem Transformator Tr, einer Diode D1 und einem Kondensator C3. Die Arbeitsfrequenz dieses, gleichfalls von der Spannungsquelle 1 gespeisten astabilen Multivibrators 12 mit den Transistoren T3 und T4 liegt oberhalb von 20 kHz, wodurch akustische Belästigungen ausgeschlossen sind und der Schalenkern des Transformators Tr die besten Übertragungseigenschaften aufweist. Die am Kollektor des Transistors T4 zur Verfügung stehende Impulsspannung wird zur Steuerung der aus Transistoren T5 und T6 in Darlingtonschaltung aufgebauten Leistungsstufe 13 verwendet. Im Kollektorstromkreis der Darlingtonschaltung liegt die Primärwicklung des Transformators Tr, dessen Sekundärwicklung einen entgegengesetzten Wicklungssinn aufweist. Der Gleichspannungswandler 3 arbeitet nach dem Sperrwandler-Prinzip, d.h. während der Impulsdauer wird aufgrund des Stromflusses durch die Primärwicklung des Transformators Tr in dessen Magnetkern Energie gespeichert, die währen der Impulspause über die Sekundärwicklung und die Diode D1 an den Kondensator C3 abgegeben wird. Die Ausgangsspannung des Gleichspannungswandlers 3 wird vom Ladezustand des Kondensators C3 bestimmt. Der elektrische Schalter 4 besteht aus zwei Transistoren T7 und T8 in Darlingtonschaltung, wobei die Basis des Transistors T7 an den Ausgang des Impulserzeugers 2 und die Kollektoren der Transistoren T7; T8 an die Verbindungsleitung zwischen der Diode D1 und den Kondensator C3 des Gleichspannungswandlers 3 geschaltet sind. Sobald die Transistoren T7; T8 leitend werden, entlädt sich der Kondensator C3.

Während die Entladung des Kondensators C3 über eine zwischen die Ausgangsbuchse 6a für die Ösophaguselektrode und den Kondensator C3 geschaltete Diode D2 und einen Widerstand R7, den Patientenwiderstand, den elektronischen Schalter 4 sowie über den parallel zur Reihenschaltung aus Widerstand R7 und Patientenwiderstand liegenden Widerstand R8 erfolgt, wird der Kondensator C3 über eine weitere Diode D3 aufgeladen, die mit dem gleichen Belag des Kondensators C3 wie die Diode D2 verbunden, jedoch entgegengesetzt gepolt ist. Ein zur Diode D3 parallel geschalteter Kondensator C4 kompensiert den Innenwiderstand dieser Diode D3 für hohe Frequenzen. Damit wird erreicht, daß der Spannungsanteil der Gleichspannungswandlerfrequenz am Eingang zu der aus Diode D2 und Widerstand R7 gebildeten Sicherheitsschaltung 5 vernachlässigbar ist. Die Diode D2 verhindert ein Aufladen des Kondensators C3 über den Patienten. Der Widerstand R7 dient zum Schutz des elektronischen Schalters 4 bei einem Kurzschluß zwischen den Ausgangsbuchsen 6a und 6b bzw. der Ösophaguselektrode z.B. bei Ablage derselben auf einer leitfähigen Unterlage.

Die Signalisierungsschaltung 7 besteht aus der Reihenschaltung eines Widerstandes R12, einer Leuchtdiode D4 une eines Transistors T15, wobei die Basis des Transistors T15 über einen Widerstand R13 an den Ausgang des Impulserzeugers 2 geschaltet ist.

Die Ladespannung am Kondensator C3 wird mit Hilfe eines Regelkreises stabilisiert, indem sie dem Schwellwertschalter 9 zugeführt wird, der aus einer Reihenschaltung einer auf eine Fotodiode D10 arbeitenden Leuchtdiode D5 und mehreren Zenerdioden D6 besteht, wobei die entsprechend der zu stabilisierenden Spannung nicht benötigten Zenerdioden mit Hilfe des Stufenschalters S1/2 kurzschließbar sind. Als Leuchtdiode D5 und Fotodiode D10 kann ein üblicher Optokoppler verwendet werden. Der Ausgang des Schwellwertschalters 9 ist über einen als Verstärker arbeitenden Transistor T9 an den set-Eingang des bistabilen Multivibrators 10 geschaltet. Der reset-Eingang des mit den Transistoren T10 und T11 ausgerüsteten bistabilen Multivibrators 10 ist hingegen mit dem Ausgang des Impulserzeugers 2 verknüpft, wodurch der bistabile Multivibrator 10 am Ende jedes Stimulationsimpulses zurückgesetzt wird. Sobald die Ladespannung am Kondensator C3 den eingestellten Schwellwert überschreitet, d.h. durch die Leuchtdiode D5 ein Strom fließt und damit der Transistor T9 leitend wird, schaltet der bistabile Multivibrator 10 in seinen zweiten stabilen Zustand und schließt über einen Transistor T12 den Ausgang des astabilen Multivibrators 12 gegen Masse kurz, was bewirkt, daß keine Leistung mehr übertragen wird. Diese Art Regelung der Ladespannung am Kondensator C3 hat gegenüber einer stetigen Regelung durch Änderung des Tastverhältnisses der Gleichspannungswandlerfrequenz mit Hilfe eines mit dem astabilen Multivibrators 12 verknüpften Dämpfungsgliedes den Vorteil, daß die für den Transformator Tr günstigste Frequenz und das optimale Tastverhältnis angewendet werden kann.

Um ein unzulässiges Anwachsen der Ladespannung am Kondensator C3 bei einem Defekt einer der Zenerdioden D6 zu vermeiden, ist zur Spannungsbegrenzung als Überlastungsschutzschaltung 11 der Zenerdiodenkette D6 eine zweite Reihenschaltung von Zenerdioden D7 mit einer höheren Schwellspannung parallel geschaltet.

Die Signalisierungsschaltung 8 zur Anzeige des Ladezustandes der Spannungsquelle 1 ist als Blinkschaltung aufgebaut. Zu diesem Zweck wird die Gleichspannung der Spannungsquelle 1 der Reihenschaltung eines Widerstandes R9 und einer Zenerdiode D8 zugeführt. Parallel zu dieser Reihenschaltung liegen in Reihe ein Widerstand R10, eine Leuchtdiode D9 und eine weiterer Widerstand R11. An den Verbindungspunkt zwischen Widerstand R9 und Zenerdiode D8 ist der Emitter eines Transistors T13 und an den Verbindungspunkt zwischen Leuchtdiode D9 und Widerstand R11 die Basis des Transistors T13 geschaltet. Der Kollektor des Transistors T13 ist mit der Basis eines weiteren Transistors T14 verbunden, dessen Kollektor-Emitter-Strecke parallel zum hochohmigen Widerstand R11 geschaltet ist. Liegt an der Basis des Transistors T13 annähernd die für den Betrieb der Stimulationseinrichtung erforderliche Gleichspannung der Spannungsquelle 1, so sind die Transistoren T13 und T14 gespert und durch die Leichtdiode D9 fließt nur ein geringer Strom. Sinkt hingegen die Gleichspannung der Spannungsquelle 1 und kommt in den Bereich der Zenerspannung der Zenerdiode D8, so werden die Transistoren T13 und T14 leitend, ein der Zenerdiode D8 parallel geschalteter Kondensator C5 entladt sich und die Leuchtdiode D9 leuchtet. Sobald der Kondensator C5 entladen ist, sperren die Transistoren T13 und T14 wieder. Der Kondensator C5 lädt sich erneut auf, die Transistoren T13 und T14 werden wieder leitend und die Leuchtdiode D9 leuchtet. Je kleiner die Gleichspannung der Spannungsquelle 1 wird, desto schneller ist die Blinkfolge. Diese Art Signalisierungsschaltung 8 hat den Vorteil, daß sie mit einem geringen Ruhestrom behaftet ist.

## Patentansprüche

1. Einrichtung zur elektrischen Stimulation des Herzens mit einem elektronischen Impulserzeuger (2) und einer Reizelektrode, insbesondere einer Ösophaguselektrode, gekennzeichnet durch einen aus einem astabilen Multivibrator (12), einer Leistungsstufe (13), einem Transformator (Tr), einem Gleichrichter (D1) und einem Kondensator (C3) bestehenden und eingangsseitig an eine ggf. auch den Impulserzeuger (2) speisende Spannungsquelle (1) angeschlossenen Sperrwandler (3), durch einen durch den Impulserzeuger (2) betätigbaren elektronischen Schalter (4), der während der durch den Impulserzeuger (2) bestimmten Zeitdauer des Stimulationsimpulses die Ausgangsspannung des Sperrwandlers (3) an die Reizelektrode legt, und durch zwei Dioden (D2 und D3), von denen die eine Diode (D2) zwischen einer Seite des Kondensators (C3) und der Reizelektrode liegt und gemeinsam mit dieser die Entladung des Kondensators (C3) übernimmt, während die andere Diode (D3) mit entgegengesetzter Polung an die gleiche Seite des Kondensators (C3) angeschlossen ist und der Aufladung des Kondensators (C3) dient.

2. Einrichtung nach Anspruch 1, dadurch gekennzeichnet, daß der Impulserzeuger (2) R-Wellen-synchronisiert ist.

3. Einrichtung nach Anspruch 1, dadurch gekennzeichnet, daß der Impulserzeuger (2) bei einer natürlichen Herzaktion blockbierbar ist.

4. Einrichtung nach Anspruch 1, dadurch gekennzeichnet, daß eine erste Signalisierungsschaltung (7) vorgesehen ist, welche die Abgabe von Stimulationsimpulsen an die Reizelektrode anzeigt.

5. Einrichtung nach Anspruch 1, dadurch gekennzeichnet, daß eine zweite Signalisierungsschaltung (8) vorgesehen ist, welche den Ladezustand der Spannungsquelle (1) anzeigt.

6. Einrichtung nach Anspruch 1, dadurch gekennzeichnet, daß zur Regelung der Ladespannung am Kondensator (C3) ein Dämpfungsglied vorgesehen ist, das mit dem astabilen Multivibrator (12) verknüpft ist, derart, daß die Regelung der Ladespannung durch Änderung des Tastverhältnisses der Sperrwandlerfrequenz erfolgt.

7. Einrichtung nach Anspruch 1, dadurch gekennzeichnet, daß zur Regelung der Ladespannung am Kondensator (C3) ein Schwellwertschalter (9) und ein bistabiler Multivibrator (10) vorgesehen sind sowie zwischen den astabilen Multivibrator (12) und die Leistungsstufe (13) im Sperrwandler (3) ein UND-Glied (14) eingeschaltet ist, wobei die Ladespannung dem Schwellwertschalter (9) zugeführt ist, der set-Eingang des bistabilen Multivibrators (10) mit dem Ausgang des Schwellwertschalters (9), sein reset-Eingang mit einem Ausgang des Impulserzeugers und der Ausgang des bistabilen Multivibrators (10) mit einem Eingang des UND-Gliedes (14) verknüpft sind.

8. Einrichtung nach Anspruch 1, dadurch gekennzeichnet, daß zur Ausgangsspannungsbegrenzung eine Überlastungsschutzeinrichtung (11), z.B. eine Reihenschaltung von Zenerdioden vorgesehen ist, der eine der Ladespannung des Kondensators (C3) proportionale Größe zugeführt ist.

9. Einrichtung nach Anspruch 1, dadurch gekennzeichnet, daß zwischen die Reizelektrode und die Diode (D2) ein Widerstand (R7) geschaltet ist.

## Revendications

1. Installation pour la stimulation électrique du coeur, avec un générateur électronique d'impulsions (2) et une électrode de stimulation, notamment une électrode oesophagique, installation caractérisée par un convertisseur à blocage (3) composé par un multivibrateur astable (12), d'un étage de puissance (13), d'un transformateur (Tr), d'un redresseur (D1) et d'un condensateur (C3), et raccordé, côté entrée, à une source de tension (1) alimentant aussi éventuellement le générateur d'impulsions (2), l'installation comportant un commutateur électronique (4) susceptible d'être actionné par le générateur d'impulsions (2) et qui applique à l'électrode de stimulation la tension de sortie du convertisseur à blocage (3) pendant la durée, déterminée par le générateur d'impulsions (2) de l'impulsion de stimulation, l'installation comportant également deux diodes (D2 et D3) dont l'une (D2) est placée entre un côté du condensateur (C3) et l'électrode de stimulation et assure en commun avec celle-ci la décharge du condensateur (C3), tandis que l'autre diode (D3) est raccordée, avec une polarisation inverse, au même côté du condensateur (C3) et sert à la charge de ce condensateur (C3).

2. Installation selon la revendication 1, caractérisée en ce que le générateur d'impulsions (2) est synchronisé avec les ondes R du rythme cardiaque.

3. Installation selon la revendication 1, caractérisée en ce que le générateur d'impulsions (2) est susceptible de se bloquer lors d'une action naturelle du coeur.

4. Installation selon la revendication 1, caractérisée en ce qu'il est prévu un premier circuit de signalisation (7) indiquant la délivrance d'impulsions de stimulation à l'électrode de stimulation.

5. Installation selon la revendication 1, caractérisée en ce qu'il est prévu un second circuit de signalisation (8) indiquant l'état de charge de la source de tension (1).

6. Installation selon la revendication 1, caractérisée en ce que, pour la régulation de la tension de charge sur le condensateur (C3), il est prévu un organe d'amortissement relié au multivibrateur astable (12) de telle façon que la régulation de la tension de charge s'effectue par modification du taux d'impulsions de la fréquence du convertisseur à blocage.

7. Installation selon la revendication 1, caractérisé en ce que, pour la régulation de la tension de charge sur le condensateur (C3), il est prévu un commutateur à valeur de seuil (9) et un multivibrateur bistable (10) tandis qu'entre le multivibrateur astable (10) et l'étage de puissance (13) dans le convertisseur à blocage (3) est branchée une porte "ET" (14), la tension de charge étant appliquée au commutateur à valeur de seuil (9), l'entrée de mise à l'état du multivibrateur bistable (10) étant reliée à la sortie du commutateur à valeur de seuil (9), l'entrée de remise à l'état initial de ce multivibrateur bistable étant reliée à une sortie du générateur d'impulsions, et la sortie du multivibrateur bistable (10) étant reliée à une entrée de la porte "ET" (14).

8. Installation selon la revendication 1, caractérisée en ce que, pour limiter la tension de sortie, il est prévu une installation de protection (11) contre les surcharges, par exemple un branchement en série de diodes de Zener, installation de protection à laquelle est appliquée une grandeur proportionnelle à la tension de charge du condensateur (C3).

9. Installation selon la revendication 1, caractérisée en ce qu'une résistance (R7) est branchée entre l'électrode de stimulation et la diode (D2).

**Claims**

1. A device for electrical stimulation of the heart, comprising an electronic pulse generator (2) and an active electrode, especially an oesophagial electrode, characterized by an inhibit transducer (3) formed of an astable multivibrator (12), a power circuit (13), a transformer (Tr), a rectifier (D1), and a capacitor (C3), the input of said inhibit transducer (3) being connected to a voltage source (1) which possibly also supplies the pulse generator (2), and by an electronic switch (4) which is actuated by the pulse generator (2) and which, during the period of the stimulation pulse as determined by the pulse generator (2), applies the output voltage of the inhibit transducer (3) to the active electrode, and by two diodes (D2 and D3) of which one diode (D2) is connected between one side of the capacitor (C3) and the active electrode and together therewith provides for discharge of the capacitor (C3), whereas the other diode (D3) is connected with opposite polarity to the same side of the capacitor (C3) and serves to charge the capacitor (C3).

2. A device as claimed in claim 1, characterized in that the pulse generator (2) is R-wave synchronized.

3. A device as claimed in claim 1, characterized in that upon occurrence of a natural cardiac action the pulse generator (2) may be inhibited.

4. A device as claimed in claim 1 characterized in that a first signaling circuit (7) is provided which signals the emission of stimulation pulses to the active electrode.

5. A device as claimed in claim 1, characterized in that a second signaling circuit (8) is provided which signals the condition of charge of the voltage source (1).

6. A device as claimed in claim 1, characterized in that an attenuator element is provided for controlling the charging voltage across the capacitor (C3), said attenuator element being combined to the astable multivibrator (12) such that the charging voltage is controlled by varying the duty cycle of the inhibit transducer frequency.

7. A device as claimed in claim 1, characterized in that for controlling the charging voltage across the capacitor (C3) there are provided a threshold switch (9) and a bistable multivibrator (10), and an AND-circuit (14) is connected between the astable multivibrator (12) and the power circuit (13) in the inhibit transducer (3), the charging voltage being supplied to the threshold switch (9), the set input of the bistable multivibrator (10) being connected to the output of the threshold switch (9), the reset input thereof being connected to an output of the pulse generator, and the output of the bistable multivibrator (10) being connected to an input of the AND circuit (14).

8. A device as claimed in claim 1, characterized in that for limiting the output voltage there is provided an overload protection means (11), e.g. a series connection of Zener diodes, to which a value proportional to the charging voltage of the capacitor (C3) is supplied.

9. A device as claimed in claim 1, characterized in that a resistor (R7) is connected between the active electrode and the diode (D2).

Fig.1

0029 479

Fig.2

0029 479